# FASCICULE DE BREVET EUROPEEN

(11) **EP 0 742 003 B1**
(45) Date de publication et mention de la délivrance du brevet: **03.09.1997**
(21) Numéro de dépôt: 96400801.5
(22) Date de dépôt: 15.04.1996
(51) Int. Cl.: A61K 7/42

(54) **Compositions cosmétiques pour la photoprotection de la peau et/ou des cheveux à base d'un mélange synergique de filtres et utilisations**
Kosmetische Zusammensetzungen zum Lichtschutz der Haut und/oder der Haare auf Basis von einer synergetischen Mischung von Filtern und Verwendungen
Cosmetic composition for skin and/oder hair sun protection based on a synergistic mixture of filters and use

(30) Priorité: 12.05.1995 FR 9505677
(43) Date de publication de la demande: 13.11.1996
(73) Titulaire: L'OREAL, 75008 Paris (FR)
(72) Inventeur: Ascione, Jean-Marc, 75015 Paris (FR); Pisson, Anne-Marie, 91500 Brunoy (FR)
(74) Mandataire: Andral, Christophe André Louis

(56) Documents cités:
- WO-A-95/00111
- FR-A- 2 642 968
- FR-A- 2 695 560

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre un premier filtre particulier, à savoir l'acide benzène 1,4-di(3-méthylidène-10-camphosulfonique) avec au moins un deuxième filtre particulier convenablement sélectionné au sein des silicones benzotriazoles.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV).

A la suite d'importantes recherches menées dans le domaine de la photoprotection évoqué ci-dessus, la Demanderesse a découvert, de façon inattendue et surprenante, qu'une combinaison de deux filtres solaires particuliers et déjà connus en soi dans l'état de l'art, permettait, du fait d'un effet de synergie, d'obtenir des compositions antisolaires présentant des facteurs de protection solaire nettement améliorés.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) de l'acide benzène 1,4-di(3-méthylidène-10-camphosulfonique), éventuellement sous forme partiellement ou totalement neutralisée, à titre de premier filtre, et (ii), à titre de deuxième filtre, une silicone benzotriazole choisie parmi les silicones benzotriazoles répondant à l'une des formules suivantes: 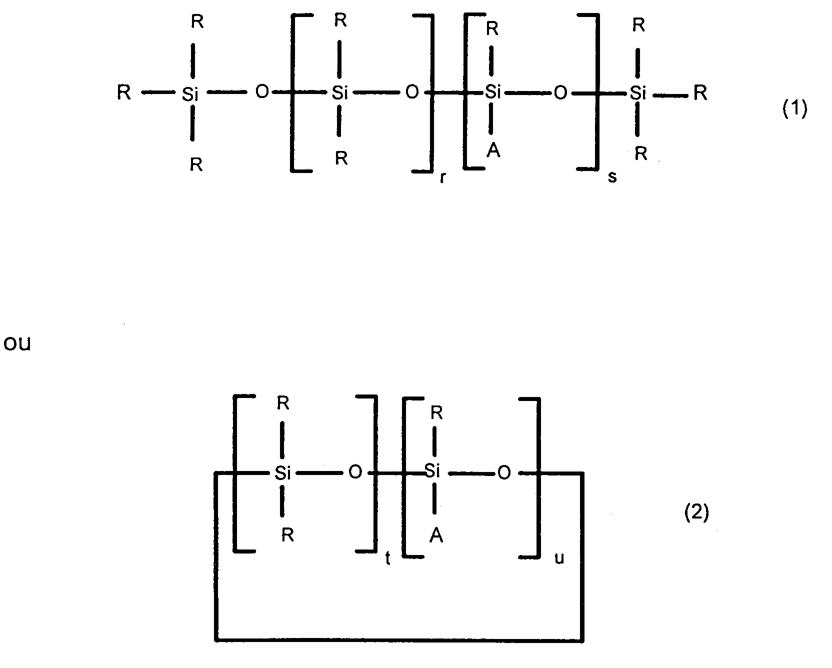 formules (1) et (2) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante : 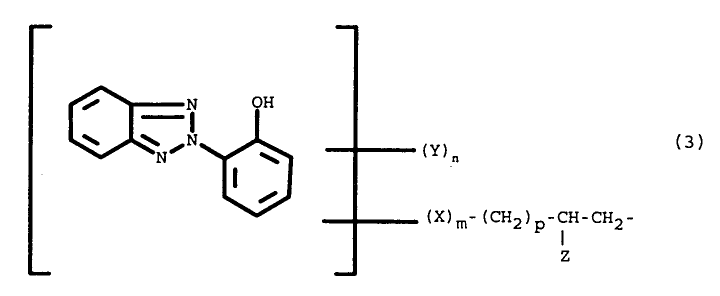 formule (3) dans laquelle :
   - Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
   - X représente O ou NH,
   - Z représente l'hydrogène ou un radical alkyle en C₁-C₄,
   - n est un nombre entier compris entre 0 et 3 inclusivement,
   - m est 0 ou 1,
   - p représente un nombre entier compris entre 1 et 10, inclusivement.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

L' acide benzène 1,4-di(3-méthylidène-10-camphosulfonique) et ses différents sels (composé (I)), décrits notamment dans les demandes de brevets FR-A- 2 528 420 et FR-A- 2 639 347, sont des filtres déjà connus en soi (filtres dits à large bande) capables en effet d'absorber les rayons ultraviolets de longueur d'ondes comprises entre 280 nm et 400 nm, avec des maxima d'absorption compris entre 320 nm et 400 nm, en particulier aux alentours de 345 nm. Ces filtres répondent à la formule générale (4) suivante : 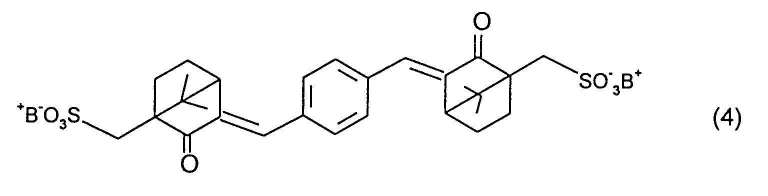 dans laquelle B désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺. Il est bien entendu que les composés de formule (4) ci-dessus peuvent donner lieu à l'isomère "cis-trans" autour d'une ou plusieurs double(s) liaison(s) et que tous les isomères rentrent dans le cadre de la présente invention.

Les silicones benzotriazoles utilisées dans le cadre de la présente invention (composé (II)) appartiennent à la famille générale des silicones benzotriazoles qui est décrite notamment dans FR-A-2 642 968. Comme indiqué précédemment, les silicones benzotriazoles sélectionnées selon l'invention sont celles qui répondent aux formules suivantes: 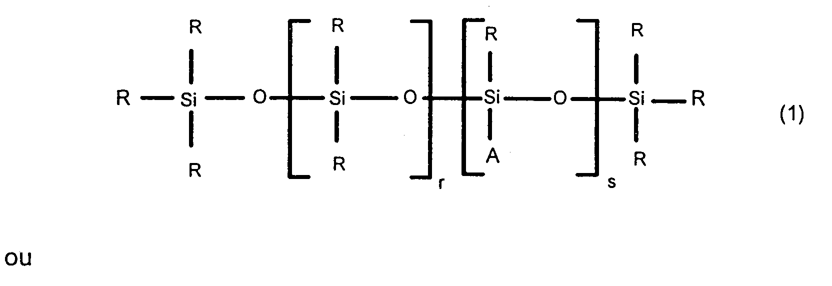 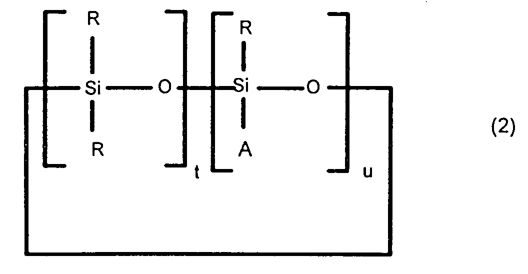 formules (1) et (2) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule suivante : 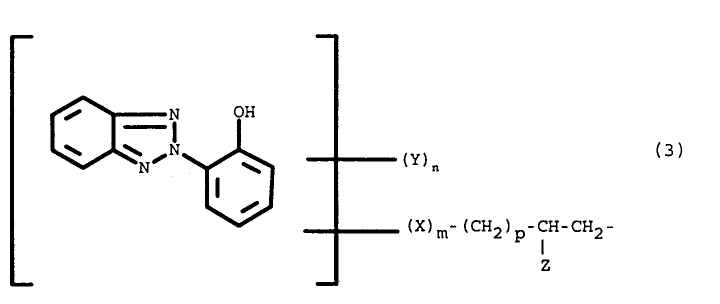 formule (3) dans laquelle :
   - Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
   - X représente O ou NH,
   - Z représente l'hydrogène ou un radical alkyle en C₁-C₄,
   - n est un nombre entier compris entre 0 et 3 inclusivement,
   - m est 0 ou 1,
   - p représente un nombre entier compris entre 1 et 10, inclusivement.

Comme cela ressort de la formule (3) donnée ci-dessus, l'accrochage du chaînon -(X)ₘ-(CH₂)ₚ-CH(Z)-CH₂- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

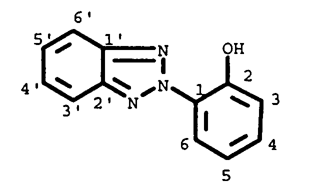

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du motif substituant Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage se fait en position 5.

Dans les formules (1) et (2) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formules (1) ou (2) ci-dessus, on préfère mettre en oeuvre ceux répondant à la formule (1), c'est-à-dire des diorganosiloxanes à chaîne courte linéaire.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement est méthyle,
- r est compris entre 0 et 15 inclusivement ; s est compris entre 1 et 5 inclusivement,
- n est non nul, et de préférence égal à 1, et Y est alors choisi parmi méthyle, ter.-butyle ou alcoxy en C₁-C₄,
- Z est hydrogène ou méthyle,
- m=0, ou [m=1 et X=O]
- p est égal à 1.

Une famille de composés convenant particulièrement à l'invention est celle définie par la formule générale suivante : 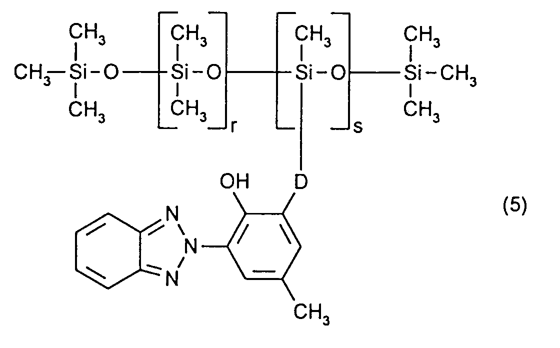
- avec: 0 ≤ r ≤ 15, de préférence 0 ≤ r ≤ 10
1 ≤ s ≤ 5 ,de préférence 1 ≤ s ≤ 3
et où D représente le radical divalent :
⁅CH₂⁆₃
ou

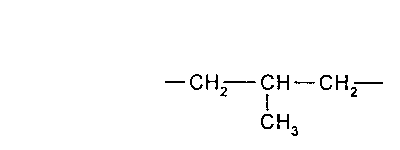

Dans une forme particulièrement préférée de réalisation de l'invention, la silicone benzotriazole répond à la formule générale (5) dans laquelle :
- r=: 0
- s=: 1
- D=:

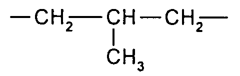

Dans une autre forme particulièrement préférée de réalisation de l'invention, la silicone benzotriazole répond à la formule générale (5) dans laquelle :
- r=: 0
- s=: 1
- D=: ⁅CH₂⁆₃

Pour préparer les filtres siliconés de formule (1) et (2), on peut procéder classiquement en mettant en oeuvre une réaction d'hydrosylilation (à savoir 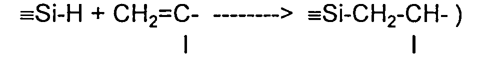 à partir de la silicone correspondante dans laquelle, par exemple, tous les radicaux A sont des atomes d'hydrogène. Cette silicone de départ est dénommée par la suite dérivé à SiH. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709. Ce dérivé à SiH peut être donc représenté soit par la formule (1 bis) suivante : 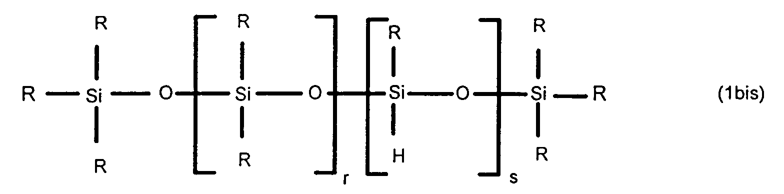 dans laquelle R, r et s ont la signification donnée ci-dessus pour la formule (1),
soit par la formule (2bis) suivante : 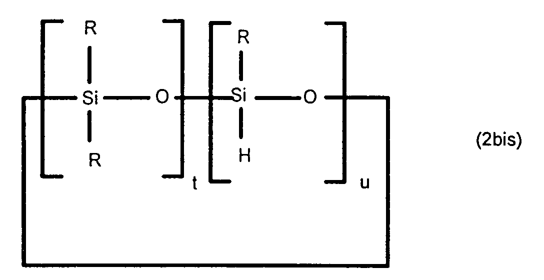 dans laquelle R, t et u ont la signification donnée ci-dessus pour la formule (2).

Sur ce dérivé à SiH de formules (1bis) ou (2bis), on effectue donc une réaction d'hydrosilylation classique, opérée en présence d'une quantité catalytiquement efficace d'un catalyseur au platine, sur un dérivé organique de benzotriazole de formule (3bis) suivante : 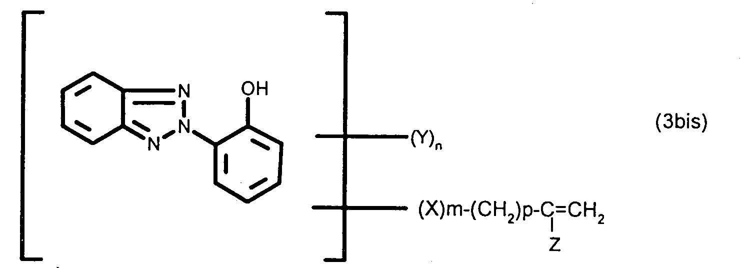 dans laquelle Y, X, Z, n, m et p ont la signification donnée ci-dessus pour la formule (3).

Des procédés convenant à la préparation des produits de formule (3bis) ci-dessus sont notamment décrits dans les brevets US- 4 316 033 et US- 4 328 346.

En outre, les détails des conditions opératoires à suivre pour conduire la réaction d'hydrosylilation entre les composés de formule (1bis) ou (2bis) ci-dessus avec le composé de formule (3bis) ci-dessus sont donnés dans la demande de brevet EP- 0 392 883, dont l'enseignement est, à cet égard, totalement inclus à titre de référence dans la présente description.

Le composé (I) peut être présent dans les compositions selon l'invention à une concentration comprise entre 0,1 et 10%, de préférence entre 0,2 et 8%, en poids par rapport au poids total de la composition et le composé (II) peut être, quant à lui, présent à des teneurs comprises entre 0,1 et 10%, de préférence entre 0,2 et 8%, en poids, toujours par rapport au poids total de la composition ; de préférence, la teneur globale du mélange entre le composé (I) et le composé (II) n'excède pas 15% du poids total de la composition finale.

D'un point de vue pratique, les deux composés (I) et (II) ci-dessus sont bien entendu de préférence tous deux présents dans la composition finale dans des proportions respectives choisies de manière telle que l'effet de synergie, au niveau du facteur de protection solaire conféré par l'association résultante, soit optimal. La plage exacte des rapports pondéraux [composé (I)/composé (II)] dans laquelle cet effet de synergie optimal est effectivement atteint peut varier légèrement selon la quantité totale de filtres (I) et (II) mise en oeuvre.

En outre, et d'une manière générale, on notera que les concentrations et rapports en composés (I) et (II) sont choisis de manière telle que le facteur de protection solaire de la composition finale soit de préférence d'au moins 2.

Enfin, toujours selon un mode préféré de réalisation de la présente invention, le support cosmétiquement acceptable dans lequel se trouvent contenus les différents composés (I) et (II) est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).
Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier l'effet de synergie, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.
Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.
Des exemples concrets, mais nullement limitatifs, illustrant l'invention, vont maintenant être donnés.

### EXEMPLE 1

On a préparé diverses formulations antisolaires se présentant sous la forme d'une émulsion de type huile-dans-eau et contenant (les quantités sont exprimées en % de poids par rapport au poids total de la composition) :

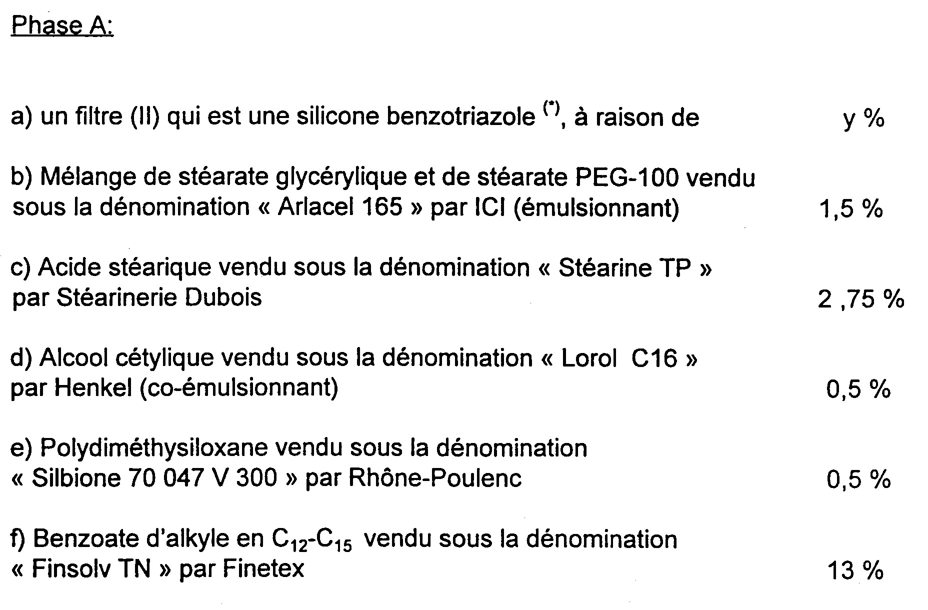

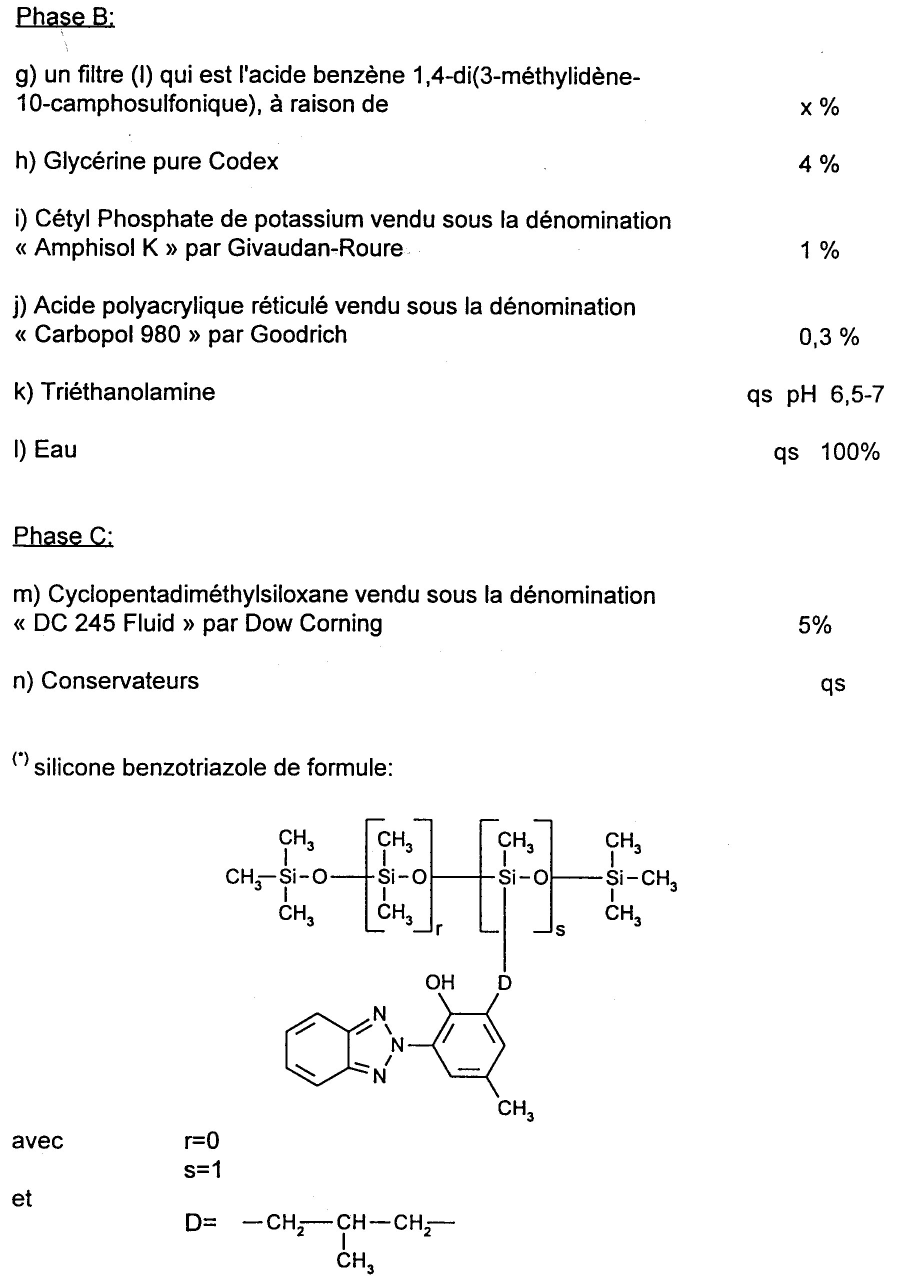

On a réalisé chacune de ces émulsions de la manière suivante: les phases A et B ont été préalablement chauffées et homogénéisées à 80°C. La phase A a ensuite été dispersée sous agitation rapide dans la phase B. On a laissé le mélange refroidir sous agitation douce. A 40°C, on a ajouté la phase C.

Pour chacune des formulations ainsi préparées, on a ensuite déterminé le facteur de protection solaire (SPF) qui lui était attaché. La mesure du facteur de protection solaire a été effectuée selon la méthode suivante (*in vivo*): on a appliqué ces formulations, à raison de 2 mg de produit / cm² de peau, sur le dos de 10 modèles humains, puis on a soumis simultanément les zones protégées et les zones non protégées de peau à l'action d'un simulateur solaire commercialisé sous le nom de "Xénon Multiport WG 320-UG 11" ; le facteur de protection solaire (SPF) a été alors calculé mathématiquement par le rapport du temps d'irradiation qui a été nécessaire pour atteindre le seuil érythématogène avec le filtre UV (zone protégée) au temps qui a été nécessaire pour atteindre le seuil érythématogène sans filtre UV (zone non protégée).

Les compositions des différentes formulations étudiées et les résultats en facteur de protection solaire moyen (moyenne sur les 10 modèles) obtenus sont rassemblés dans le tableau (I) donné ci-dessous.

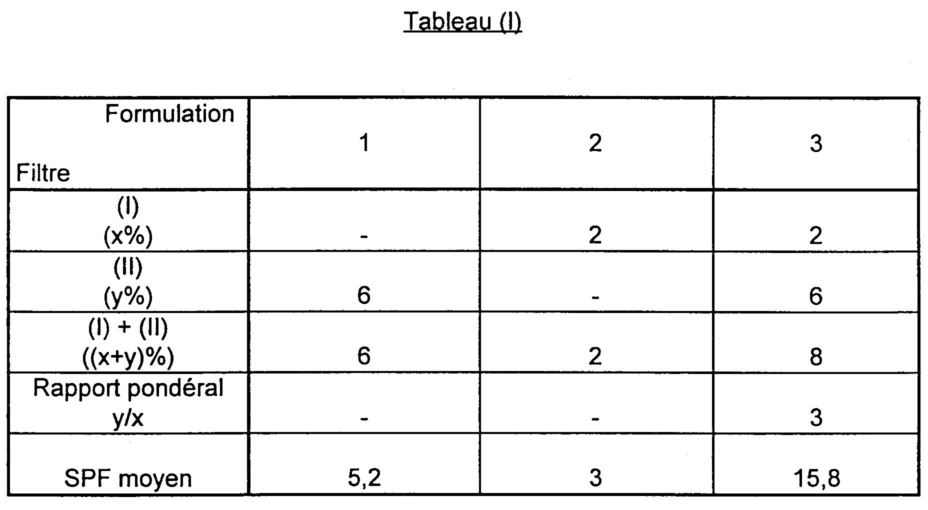

Ces résultats démontrent clairement l'effet de synergie obtenu avec la composition 3 conforme à l'invention, le facteur de protection solaire attaché à cette dernière étant notablement et significativement supérieur à la simple somme arithmétique des facteurs de protection solaire des compositions comparatives correspondantes ne contenant qu'un seul filtre.

### EXEMPLE 2:

On donne ci-dessous un exemple concret de composition antisolaire conforme à l'invention se présentant sous la forme d'une émulsion de type huile-dans-eau: les quantités sont exprimées en pourcentage en poids par rapport au poids total de la composition.

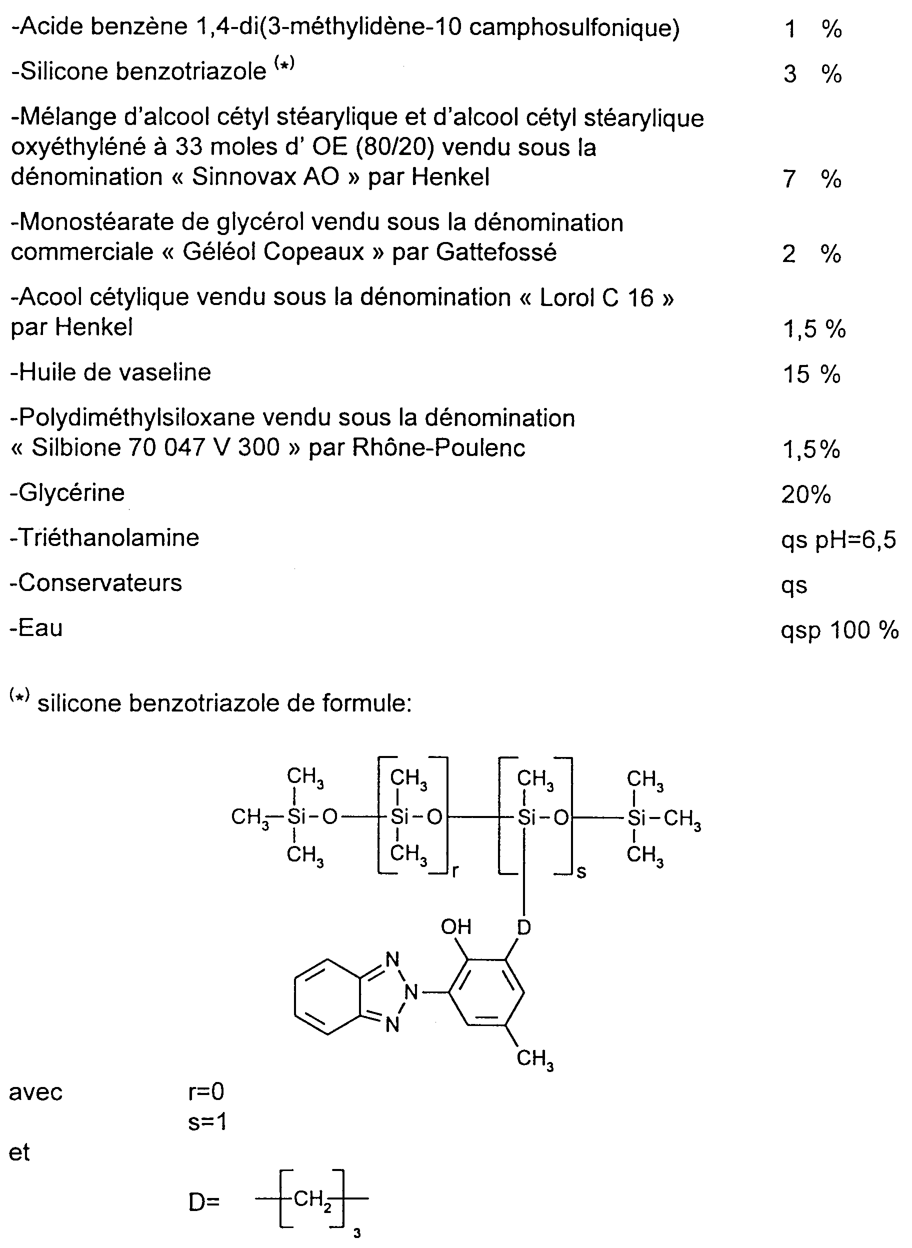

## Revendications

1. Composition cosmétique à usage topique, en particulier pour la photoprotection de la peau et/ou des cheveux, caractérisée par le fait qu'elle comprend, dans un support cosmétiquement acceptable, (i) de l'acide benzène 1,4-di(3-méthylidène-10-camphosulfonique), éventuellement sous forme partiellement ou totalement neutralisée, à titre de premier filtre, et, (ii) à titre de deuxième filtre, une silicone benzotriazole choisie parmi les silicones benzotriazoles répondant aux formules suivantes: 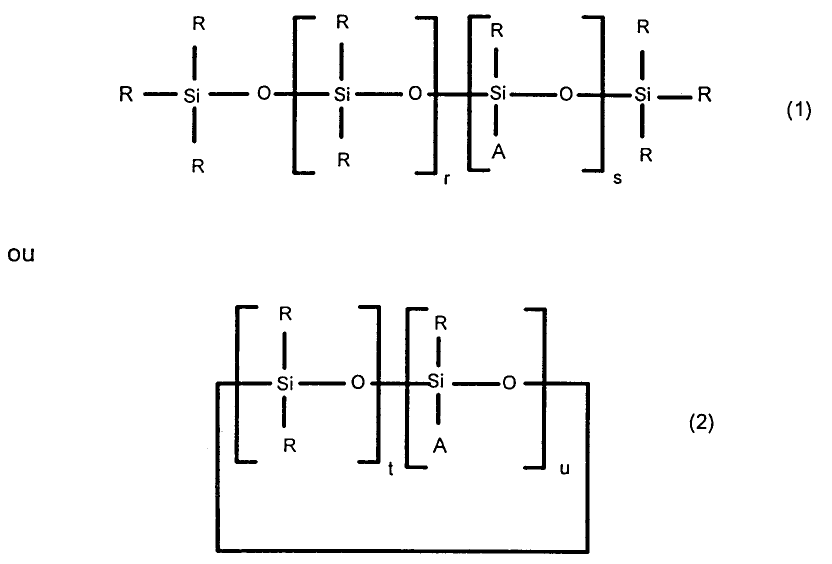 formules (1) et (2) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante : 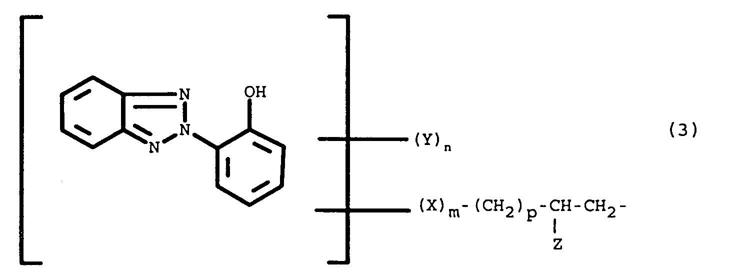 formule (3) dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.

2. Composition selon la revendication 1, caractérisée par le fait que lesdits premier et second filtres sont pésents dans ladite composition dans une proportion produisant une activité synergique.

3. Composition selon les revendications 1 ou 2, caractérisée par le fait que la silicone benzotriazole est choisie parmi les composés de formule générale (1) présentant au moins l'une des caractéristiques suivantes :
- R est alkyle, et de préférence méthyle,
- r est compris entre 0 et 15 inclusivement ; s est compris entre 1 et 5 inclusivement,
- n est non nul, et de préférence égal à 1, et Y est alors choisi parmi méthyle, ter.-butyle ou alcoxy en C₁-C₄,
- Z est hydrogène ou méthyle,
- m=0, ou [m=1 et X=O]
- p est égal à 1.

4. Composition selon la revendication 3, caractérisée par le fait que ledit composé de formule générale (1) présente l'ensemble desdites caractéristiques.

5. Composition selon la revendication 4, caractérisée par le fait que la silicone benzotriazole est choisie parmi les composés de formule générale (5): 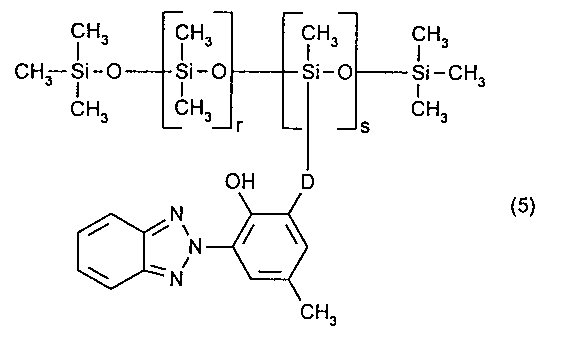
avec 0 ≤ r ≤ 15
1 ≤ s ≤ 5
et où D représente le radical divalent :
⁅CH₂⁆₃
ou

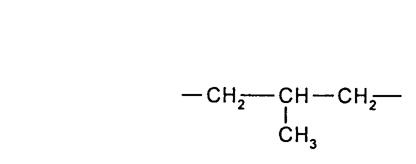

6. Composition selon la revendication 5, caractérisée par le fait que le deuxième filtre est la silicone benzotriazole répondant à la formule générale (5) dans laquelle :
r= 0
s= 1
et
D=

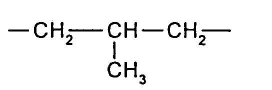

7. Composition selon la revendication 5, caractérisée par le fait que le deuxième filtre est la silicone benzotriazole répondant à la formule générale (5) dans laquelle :
r= 0
s= 1
et
D= ⁅CH₂⁆₃

8. Composition selon l'une quelconque des revendications précédentes, caractérisée par le fait que ledit premier filtre répond à la formule (4) suivante : 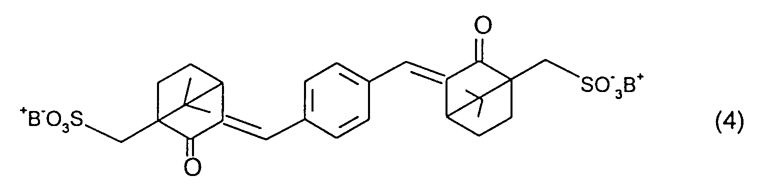 dans laquelle B désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺.

9. Composition selon l'une quelconque des revendications précédentes, caractérisée par le fait que la concentration en premier filtre est comprise entre 0,1 et 10 % en poids par rapport au poids total de la composition.

10. Composition selon la revendication 9, caractérisée par le fait que ladite concentration est comprise entre 0,2 et 8 % en poids par rapport au poids total de la composition.

11. Composition selon l'une quelconque des revendications précédentes, caractérisée par le fait que la concentration en deuxième filtre est comprise entre 0,1 et 10 % en poids par rapport au poids total de la composition.

12. Composition selon la revendication 11, caractérisée par le fait que ladite concentration est comprise entre 0,2 et 8 % en poids par rapport au poids total de la composition.

13. Composition selon l'une quelconque des revendications précédentes, caractérisée par le fait que ledit support cosmétiquement acceptable se présente sous la forme d'une émulsion de type huile-dans-eau.

14. Composition selon l'une quelconque des revendications précédentes, caractérisée par le fait qu'elle comprend en outre un ou plusieurs filtres organiques complémentaires actifs dans l'UV-A et/ou UV-B, hydrophiles ou lipophiles, différents desdits premier et deuxième filtres.

15. Composition selon la revendication 14, caractérisée par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β -diphénylacrylate, les dérivés de l'acide p-aminobenzoïque, les polymères filtres et silicones filtres.

16. Composition selon l'une quelconque des revendications précédentes, caractérisée par le fait qu'elle comprend en outre, à titre d'agents photoprotecteurs UV complémentaires, des pigments ou des nanopigments d'oxydes métalliques, enrobés ou non.

17. Composition selon la revendication 16, caractérisée par le fait que lesdits pigments ou nanopigments sont choisis parmi les oxydes de titane, de zinc, de fer, de zirconium, de cérium et leurs mélanges, enrobés ou non.

18. Composition selon l'une quelconque des revendications précédentes, caractérisée par le fait qu'elle comprend en outre au moins un agent de bronzage et/ou de brunissage artificiel de la peau.

19. Composition selon l'une quelconque des revendications précédentes, caractérisée par le fait qu'elle comprend en outre au moins un adjuvant choisi parmi les corps gras, les solvants organiques, les épaississants ioniques ou non ioniques, les adoucissants, les antioxydants, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

20. Composition selon l'une quelconque des revendications précédentes, caractérisée par le fait qu'il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'un bâtonnet solide, d'une mousse ou d'un spray.

21. Composition selon l'une quelconque des revendications 1 à 19, caractérisée par le fait qu'il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

22. Composition selon l'une quelconque des revendications 1 à 19, caractérisée par le fait qu'il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique ou d'une laque pour cheveux.

23. Composition selon l'une quelconque des revendications précédentes, caractérisée par le fait qu'elle présente un facteur de protection solaire sur peau d'au moins 2.

24. Utilisation de la composition définie à l'une quelconque des revendications précédentes comme, ou pour la fabrication de, compositions cosmétiques pour la protection de la peau et/ou des cheveux contre le rayonnement ultraviolet, en particulier le rayonnement solaire.

25. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 23.

## Claims

1. Cosmetic composition for topical use, in particular for the photoprotection of the skin and/or of the hair, characterized in that it comprises, in a cosmetically acceptable carrier, (i) 1,4-benzene[di(3-methylidene-10-camphosulphonic)] acid, optionally in a partially or completely neutralized form, as first screening agent, and (ii) as second screening agent, a benzotriazole silicone chosen from the benzotriazole silicones corresponding to the following formulae: 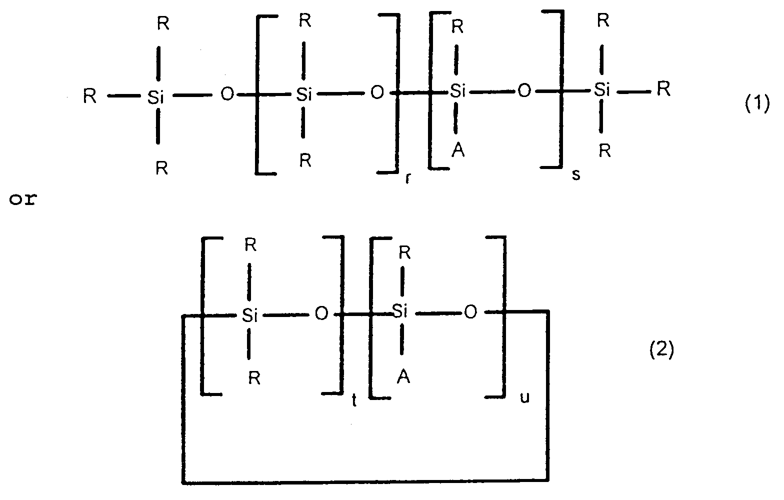 in which formulae (1) and (2):
- R, which are identical or different, are chosen from C₁-C₁₀ alkyl, phenyl or 3,3,3-trifluoropropyl radicals, at least 80 % in numerical terms of the R radicals being methyl,
- r is a integer between 0 and 50 inclusive, and s is a integer between 1 and 20 inclusive,
- u is an integer between 1 and 6 inclusive, and t is an integer between 0 and 10 inclusive, it being understood that t + u is equal to or greater than 3,
- and the symbol A designates a monovalent radical attached directly to a silicon atom, and which corresponds to the following formula (3): 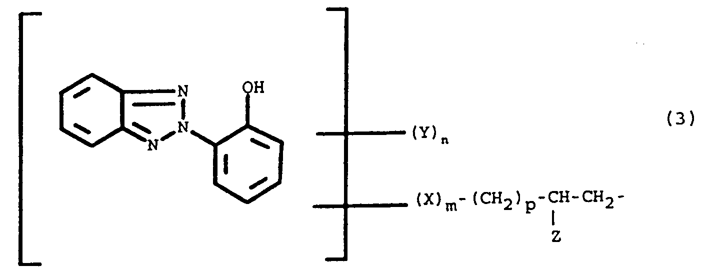 in which formula (3):
- Y, which are identical or different, are chosen from C₁-C₈ alkyl radicals, halogen atoms and C₁-C₄ alkoxy radicals, it being understood that, in the latter case, two adjacent Y's of the same aromatic nucleus can form together an alkylidenedioxy group in which the alkylidene group contains from 1 to 2 carbon atoms,
- X represents O or NH,
- Z represents hydrogen or a C₁-C₄ alkyl radical,
- n is an integer between 0 and 3 inclusive,
- m is 0 or 1,
- p represents an integer between 1 and 10 inclusive.

2. Composition according to Claim 1, characterized in that the said first and second screening agents are present in the said composition in a proportion which produces a synergistic activity.

3. Composition according to Claim 1 or 2, characterized in that the benzotriazole silicone is chosen from the compounds of general formula (1) having at least one of the following characteristics:
- R is alkyl, and preferably methyl,
- r is between 0 and 15 inclusive; s is between 1 and 5 inclusive,
- n is not zero, and is preferably equal to 1, and Y is, in this case, chosen from methyl, tert-butyl or a C₁-C₄ alkoxy,
- Z is hydrogen or methyl,
- m = 0, or [m = 1 and X = O]
- p is equal to 1.

4. Composition according to Claim 3, characterized in that the said compound of general formula (1) has all the said characteristics.

5. Composition according to Claim 4, characterized in that the benzotriazole silicone is chosen from the compounds of general formula (5): 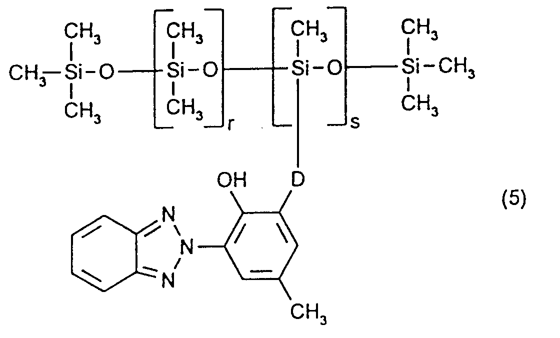
with 0 ≤ r ≤ 15
1 ≤ s < 5
and where D represents the divalent radical:
⁅CH₂⁆₃
or

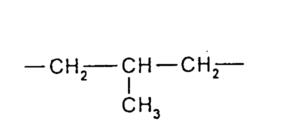

6. Composition according to claim 5, characterized in that the second screening agent is the benzotriazole silicone corresponding to the general formula (5) in which:
r= 0
s= 1
and
D=

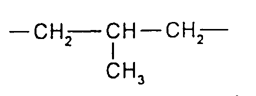

7. Composition according to Claim 5, characterized in that the second screening agent is the benzotriazole silicone corresponding to the general formula (5) in which:
r= 0
s= 1
and
D= ⁅CH₂⁆₃

8. Composition according to any one of the preceding claims, characterized in that the said first screening agent corresponds to the following formula (4): 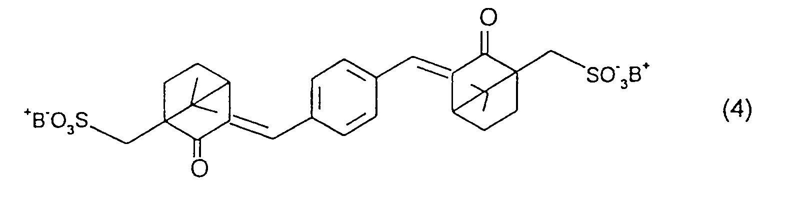 in which B designates a hydrogen atom, an alkali metal or alternatively a radical NH(R)₃⁺ in which the R radicals, which may be identical or different, designate a hydrogen atom, a C₁-C₄ alkyl or hydroxyalkyl radical or alternatively a group Mⁿ⁺/n, Mⁿ⁺ designating a polyvalent metal cation in which n is equal to 2 or 3 or 4, Mⁿ⁺ preferably designating a metal cation chosen from Ca²⁺, Zn²⁺, Mg²⁺, Ba²⁺, Al³⁺ and Zr⁴⁺.

9. Composition according to any one of the preceding claims, characterized in that the concentration of the first screening agent is between 0.1 and 10 % by weight relative to the total weight of the composition.

10. Composition according to Claim 9, characterized in that the said concentration is between 0.2 and 8 % by weight relative to the total weight of the composition.

11. Composition according to any one of the preceding claims, characterized in that the concentration of the second screening agent is between 0.1 and 10 % by weight relative to the total weight of the composition.

12. Composition according to Claim 11, characterized in that the said concentration is between 0.2 and 8 % by weight relative to the total weight of the composition.

13. Composition according to any one of the preceding claims, characterized in that the said cosmetically acceptable carrier is provided in the form of an oil-in-water type emulsion.

14. Composition according to any one of the preceding claims, characterized in that it comprises, in addition, one or more additional hydrophilic or lipophilic organic screening agents active in UV-A and/or UV-B, different from the said first and second screening agents.

15. Composition according to Claim 14, characterized in that the said additional organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

16. Composition according to any one of the preceding claims, characterized in that it comprises, in addition, as additional UV photoprotective agents, pigments or nanopigments of metal oxides, coated or otherwise.

17. Composition according to Claim 16, characterized in that the said pigments or nanopigments are chosen from titanium, zinc, iron, zirconium and cerium oxides and mixtures thereof, coated or otherwise.

18. Composition according to any one of the preceding claims, characterized in that it comprises, in addition, at least one artificial skin tanning and/or browning agent.

19. Composition according to any one of the preceding claims, characterized in that it comprises, in addition, at least one adjuvant chosen from fatty substances, organic solvents, ionic or nonionic thickeners, demulcents, antioxidants, opacifiers, stabilizers, emollients, silicones, α-hydroxy acids, anti-foaming agents, moisturizing agents, vitamins, perfumes, preservatives, surfactants, fillers, sequestrants, polymers, propellants, alkalinizing or acidifying agents, colorants.

20. Composition according to any one of the preceding claims, characterized in that it is a composition for protecting the human epidermis or an anti-sun composition and in that it is provided in the form of a nonionic vesicular dispersion, an emulsion, in particular an oil-in-water type emulsion, a cream, a milk, a gel, a gel cream, a suspension, a dispersion, a solid stick, a foam or a spray.

21. Composition according to any one of Claims 1 to 19, characterized in that it is a make-up composition for the eyelashes, the eyebrows or the skin and in that it is provided in a solid or pasty, anhydrous or aqueous form, an emulsion, a suspension or a dispersion.

22. Composition according to any one of Claims 1 to 19, characterized in that it is a composition intended for protecting the hair against ultraviolet rays and in that it is provided in the form of a shampoo, a lotion, a gel, an emulsion, a nonionic vesicular dispersion or a lacquer for the hair.

23. Composition according to any one of the preceding claims, characterized in that it has a sun protection factor on the skin of at least 2.

24. Use of the composition defined in any one of the preceding claims as, or for the manufacture of, cosmetic compositions for protecting the skin and/or the hair against ultraviolet radiation, in particular solar radiation.

25. Process of cosmetic treatment for protecting the skin and/or the hair against ultraviolet radiation, in particular solar radiation, characterized in that it consists in applying to them an effective quantity of a composition as defined in any one of Claims 1 to 23.

## Patentansprüche

1. Kosmetische Zusammensetzung zur topischen Anwendung und insbesondere zum Lichtschutz der Haut und/oder der Haare,
**dadurch gekennzeichnet, daß**
sie in einem kosmetisch akzeptablen Träger (i) Benzol-1,4-di(3-methylencampher-10-sulfonsäure), gegebenenfalls in teilweise oder vollständig neutralisierter Form, als erstes Filter und (ii) als zweites Filter ein Benzotriazolsilicon enthält, das unter den Benzotriazolsiliconen der folgenden Formeln ausgewählt ist: 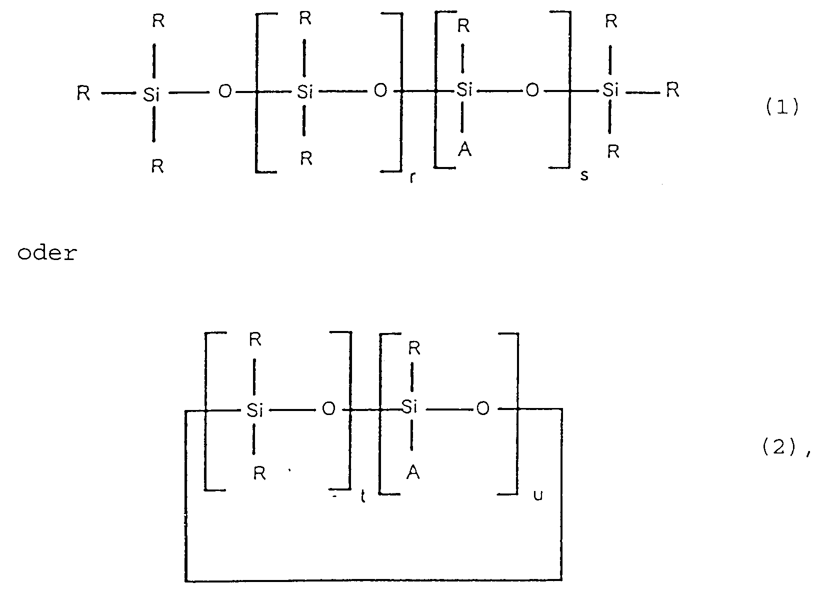 wobei in den Formeln (1) und (2):
- die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Anzahl der Gruppen R Methyl bedeuten,
- r eine ganze Zahl im Bereich von Null bis einschließlich 50, und s eine ganze Zahl im Bereich von 1 bis einschließlich 20 bedeutet,
- u eine ganze Zahl im Bereich von 1 bis einschließlich 6, und t eine ganze Zahl im Bereich von Null bis einschließlich 10 bedeutet, mit der Maßgabe, daß t + u mindestens 3 ist, und
- die Gruppe A eine einwertige Gruppe bedeutet, die direkt an ein Siliciumatom gebunden ist, und der folgenden Formel (3) entspricht: 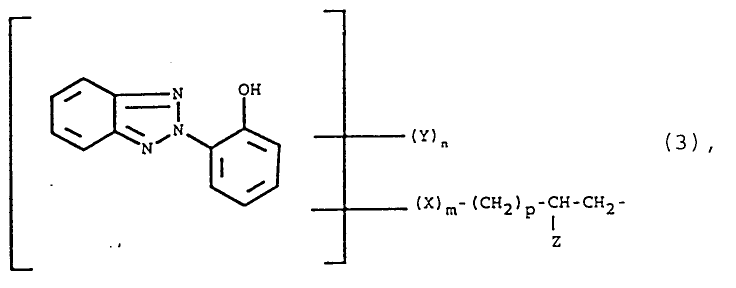 wobei in der Formel (3):
- die Gruppen Y, die identisch oder voneinander verschieden sind, unter den C₁₋₈-Alkylgruppen, Halogenen und C₁₋₄-Alkoxygruppen ausgewählt sind, mit der Maßgabe, daß im letzteren Fall zwei aneinandergrenzende Gruppen Y eines aromatischen Rings gemeinsam eine Alkylidendioxygruppe bilden können, worin die Alkylidengruppe 1 bis 2 Kohlenstoffatome enthält,
- X O oder NH bedeutet,
- Z Wasserstoff oder C₁₋₄-Alkyl bedeutet,
- n eine ganze Zahl im Bereich von Null bis einschließlich 3 bedeutet,
- m Null oder 1 ist, und
- p eine ganze Zahl im Bereich von 1 bis einschließlich 10 bedeutet.

2. Zusammensetzung nach Anspruch 1, dadurch gekennzeichnet, daß das erste und zweite Filter in der Zusammensetzung in einem Verhältnis vorliegen, das eine synergistische Wirkung hervorruft.

3. Zusmmensetzung nach Anspruch 1 oder 2, dadurch gekennzeichnet, daß das Benzotriazolsilicon unter den Verbindungen der allgemeinen Formel (1) ausgewählt ist, die mindestens eine der folgenden Eigenschaften aufweisen:
- R bedeutet Alkyl und vorzugsweise Methyl,
- r liegt im Bereich von Null bis einschließlich 15; s im Bereich von 1 bis einschließlich 5,
- n ist nicht Null und vorzugsweise 1, und Y ist unter Methyl, *tert*.-Butyl oder C₁₋₄-Alkoxy ausgewählt,
- Z bedeutet Wasserstoff oder Methyl,
- m ist Null oder [m = 1 und X = O], und
- p ist 1.

4. Zusammensetzung nach Anspruch 3, dadurch gekennzeichnet, daß die Verbindung der allgemeinen Formel (1) alle diese Eigenschaften aufweist.

5. Zusammensetzung nach Anspruch 4, dadurch gekennzeichnet, daß das Benzotriazolsilicon unter den Verbindungen der allgemeinen Formel (5) ausgewählt ist: 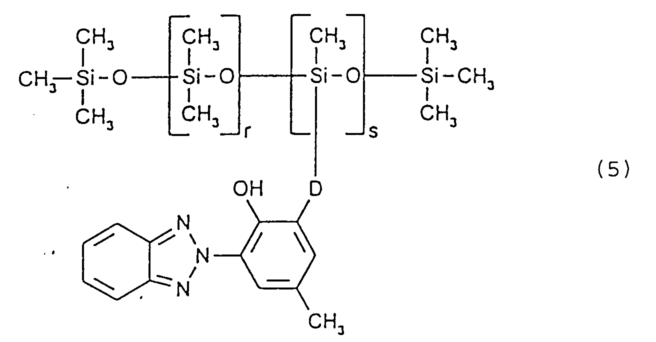
mit 0 ≤ r ≤ 15,
1 ≤ s ≤ 5,
wobei D die zweiwertige Gruppe bedeutet:
―[CH₂]₃―
oder

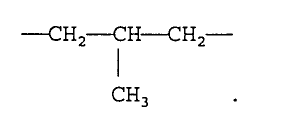

6. Zusammensetzung nach Anspruch 5, dadurch gekennzeichnet, daß das zweite Filter das Benzotriazol der allgemeinen Formel (5) ist, worin bedeuten:
r = Null,
s = 1
und
D =

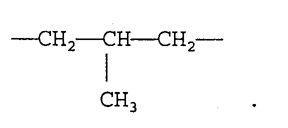

7. Zusammensetzung nach Anspruch 5, dadurch gekennzeichnet, daß das zweite Filter das Benzotriazol der allgemeinen Formel (5) ist, worin bedeuten:
r = Null,
s = 1
und
D = ―[CH₂]₃― .

8. Zusammensetzung nach einem der vorhergehenden Ansprüche, dadurch gekennzeichnet, daß das erste Filter der folgenden Formel (4) entspricht: 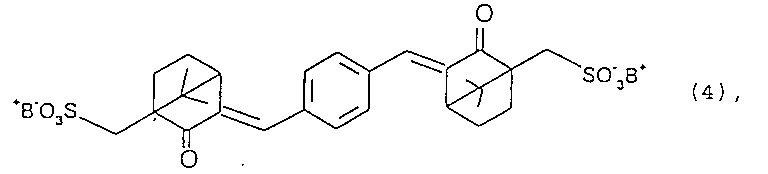 worin B Wasserstoff, ein Alkalimetall, eine Gruppe NH(R)₃⁺, worin die Gruppen R, die identisch oder voneinander verschieden sein können, Wasserstoff, Alkyl oder Hydroxyalkyl mit 1 bis 4 Kohlenstoffatomen bedeuten, oder eine Gruppe Mⁿ⁺/n bedeutet, wobei Mⁿ⁺ ein mehrwertiges Metallkation mit n gleich 2 oder 3 oder 4 ist, und wobei Mⁿ⁺ vorzugsweise ein Metallkation bedeutet, das unter Ca²⁺, Zn²⁺, Mg²⁺, Ba²⁺, Al³⁺ und Zr⁴⁺ ausgewählt ist.

9. Zusammensetzung nach einem der vorhergehenden Ansprüche, dadurch gekennzeichnet, daß die Konzentration des ersten Filters im Bereich von 0,1 bis 10 Gew.-%, bezogen auf das Gesamtgewicht der Zusammensetzung, liegt.

10. Zusammensetzung nach Anspruch 9, dadurch gekennzeichnet, daß die Konzentration im Bereich von 0,2 bis 8 Gew.-%, bezogen auf das Gesamtgewicht der Zusammensetzung, liegt.

11. Zusammensetzung nach einem der vorhergehenden Ansprüche, dadurch gekennzeichnet, daß die Konzentration des zweiten Filters im Bereich von 0,1 bis 10 Gew.-%, bezogen auf das Gesamtgewicht der Zusammensetzung, liegt.

12. Zusammensetzung nach Anspruch 11, dadurch gekennzeichnet, daß die Konzentration im Bereich von 0,2 bis 8 Gew.-%, bezogen auf das Gesamtgewicht der Zusammensetzung, liegt.

13. Zusammensetzung nach einem der vorhergehenden Ansprüche, dadurch gekennzeichnet, daß der kosmetisch akzeptable Träger in Form einer Emulsion vom Typ Öl-in-Wasser vorliegt.

14. Zusammensetzung nach einem der vorhergehenden Ansprüche, dadurch gekennzeichnet, daß sie ferner ein oder mehrere zusätzliche organische hydrophile oder lipophile Filter enthält, die im UV-A- und/oder UV-B-Bereich wirksam sind und von den ersten und zweiten Filtern verschieden sind.

15. Zusammensetzung nach Anspruch 14, dadurch gekennzeichnet, daß die zusätzlichen organischen Filter ausgewählt sind unter den Zimtsäurederivaten, Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, Polymerfiltern und Siliconfiltern.

16. Zusammensetzung nach einem der vorhergehenden Ansprüche, dadurch gekennzeichnet, daß sie ferner als zusätzliche UV-Lichtschutzmittel Pigmente oder Nanopigmente von Metalloxiden, die gegebenenfalls umhüllt sind, enthält.

17. Zusammensetzung nach Anspruch 16, dadurch gekennzeichnet, daß die Pigmente oder Nanopigmente unter Titanoxid, Zinkoxid, Eisenoxid, Zirconiumoxid, Ceroxid und deren Gemischen ausgewählt sind, wobei diese gegebenenfalls umhüllt oder nicht umhüllt sind.

18. Zusammensetzung nach einem der vorhergehenden Ansprüche, dadurch gekennzeichnet, daß sie ferner mindestens ein Mittel zum künstlichen Bräunen und/oder zur künstlichen Bräunung der Haut enthält.

19. Zusammensetzung nach einem der vorhergehenden Ansprüche, dadurch gekennzeichnet, daß sie ferner mindestens einen Hilfsstoff enthält, der ausgewählt ist unter den Fettsubstanzen, organischen Lösungsmitteln, ionischen oder nichtionischen Verdickungsmitteln, reizlindernden Mitteln, Antioxidantien, Trübungsmitteln, Stabilisierungsmitteln, Emollentien, Siliconen, α-Hydroxysäuren, Antischaummitteln, Hydratisierungsmitteln, Vitaminen, Parfums, Konservierungsmitteln, grenzflächenaktiven Stoffen, Füllstoffen, Maskierungsmitteln, Polymeren, Treibmitteln, Mitteln zum Alkalischmachen oder Ansäuern und Färbemitteln.

20. Zusammensetzung nach einem der vorhergehenden Ansprüche, dadurch gekennzeichnet, daß es sich um eine Zusammensetzung zum Schutz der menschlichen Epidermis oder ein Sonnenschutzmittel handelt, und daß die Zusammensetzung in Form einer nichtionischen Vesikeldispersion, Emulsion und insbesondere Emulsion vom Typ Öl-in-Wasser, in Form einer Creme, Milch, Gel, Gel-Creme, Suspension, Dispersion, als fester Stift, Schaum oder Spray vorliegt.

21. Zusammensetzung nach einem der Ansprüche 1 bis 19, dadurch gekennzeichnet, daß es sich um eine Zusammensetzung zum Schminken der Wimpern, Augenbrauen oder der Haut handelt, und daß die Zusammensetzung in fester oder pastöser, wasserfreier oder wässeriger Form, als Emulsion, Suspension oder Dispersion vorliegt.

22. Zusammensetzung nach einem der Ansprüche 1 bis 19, dadurch gekennzeichnet, daß es sich um eine Zusammensetzung handelt, die zum Schutz der Haare gegen UV-Strahlung bestimmt ist, und daß die Zusammensetzung in Form eines Haarwaschmittels, als Lotion, Gel, Emulsion, nichtionische Vesikeldispersion oder Haarlack vorliegt.

23. Zusammensetzung nach einem der vorhergehenden Ansprüche, dadurch gekennzeichnet, daß sie auf der Haut einen Lichtschutzfaktor von mindestens 2 aufweist.

24. Verwendung der Zusammensetzungen nach einem der vorhergehenden Ansprüche als kosmetische Zusammensetzungen zum Schutz der Haut und/oder der Haare gegen UV-Strahlung und insbesondere Sonnenlicht oder zur Herstellung dieser kosmetischen Zusammensetzungen.

25. Verfahren zur kosmetischen Behandlung zum Schutz der Haut und/oder der Haare gegen UV-Strahlung und insbesondere Sonnenlicht, dadurch gekennzeichnet, daß es darin besteht, auf die Haut und/oder die Haare eine wirksame Menge einer Zusammensetzung nach einem der Ansprüche 1 bis 23 aufzutragen.
